# EUROPEAN PATENT SPECIFICATION

(11) **EP 0 650 676 B1**
(45) Date of publication and mention of the grant of the patent: **19.01.2000**
(21) Application number: 94308019.2
(22) Date of filing: 01.11.1994
(51) Int. Cl.: A45C 11/00, B65D 75/36

(54) **Packaging arrangement for contact lenses**
Verpackung für Kontaktlinsen
Dispositif d'emballage pour lentilles de contact

(30) Priority: 02.11.1993 US 146754; 10.06.1994 US 257796
(43) Date of publication of application: 03.05.1995
(73) Proprietor: JOHNSON & JOHNSON VISION PRODUCTS, INC., Jacksonville, Florida 32216 (US)
(72) Inventor: Abrams, Richard W., Jacksonville, FL 32223 (US); Larsen, Ture-Kindt, DK-2840 Holte (DK); Martin, Wallace Anthony, Orange Park, Florida 32065 (US)
(74) Representative: Mercer, Christopher Paul

(56) References cited:
- EP-A- 0 604 177
- WO-A-93/15972
- CH-A- 363 295
- GB-A- 2 237 241
- NL-A- 6 506 271
- US-A- 3 630 346
- US-A- 4 159 771
- US-A- 4 691 820

## Description

### BACKGROUND OF THE INVENTION

### 1. Field of the Invention

The present invention relates to a packaging arrangement for the containment of at least one hydrophilic contact lens in a sterile aqueous solution. More specifically, the invention pertains to a packaging arrangement wherein a plurality of disposable hydrophilic contact lenses are contained in a specific number of individual packaging arrangements collectively housed in a box-like container or carton so as to provide a specified or essentially measured supply of contact lenses for use by a consumer over a predetermined period of time.

The packaging of hydrophilic contact lenses in a sterile aqueous solution is well known in the contact lens manufacturing technology. In particular, such packaging arrangements generally consist of so-called blister packages which are employed for the storage and dispensing of the hydrophilic contact lenses by a medical practitioner or to consumer who intends to wear the contact lenses. Generally, such hydrophilic contact lenses, which may be disposable after a single wear or short-term use, are manufactured from suitable hydrophilic polymeric materials. These materials may be, amongst others, copolymers of hydroxyethyl methacrylate containing from about 20% to 90% or more of water, depending upon the polymer composition. Generally, such contact lenses must be stored in a sterile aqueous solution, usually in isotonic saline solution in order to prevent dehydration and to maintain the lenses in a ready-to-wear condition.

### 2. Discussion of the Prior Art

Heretofore, contact lens manufacturers normally utilized stoppered glass bottles containing sterile saline solutions in which the hydrophilic contact lenses were immersed as storage and shipping containers for individual contact lenses. Each bottle was sealed with a suitable silicone stopper and provided with a metal closure as a safety seal in the configuration of an overcap. When the contact lens was intended to be removed from the bottle for use by a patient, the metal closure safety seal was required to be initially torn off the bottle, thereafter the stopper withdrawn and the lens lifted out from the bottle through the intermediary of a suitable plastic tweezer or pouring the contents out. This entailed the implementation of an extremely complicated procedure, since the contact lens was difficult to grasp and remove from the saline solution contained in the bottle due to the transparent nature of the contact lens which rendered it practically invisible to the human eye.

More recently, containments in the form of blister packages have been developed for hydrophilic contact lenses, and which enable the storage and shipping of the hydrophilic contact lenses in a simple and inexpensive expedient manner, while concurrently facilitating the conveniently easy removal of the contact lens by a practitioner or a patient.

For instance, a blister package which is adapted to provide a sterile sealed storage environment for a disposable or single-use hydrophilic contact lens, wherein the lens is immersed in a sterile aqueous solution; for example, such as in an isotonic saline solution, is described in U.S. Patent No. 4,691,820 to Martinez; which is assigned to the common assignee for the present invention.

Thus, in the above-mentioned U.S. patent, the blister package for storing and dispensing a hydrophilic contact lens includes an injection-molded or thermoformed plastic base portion incorporating a molded cavity which is surrounded by an outstanding planar flange about the rim of the cavity. A flexible cover sheet is adhered to the surface of the flange so as to sealingly enclose the cavity in a generally liquid-tight mode. Within the cavity of the base portion, a hydrophilic contact lens is immersed in a sterile aqueous solution, such as an isotonic saline solution. A portion of the side wall of the cavity is inclined to form a ramp extending upwardly towards the flange from the bottom of the cavity, and the cover sheet is adapted to be stripped from the flange in order to expose the cavity and inclined side wall whereupon the lens may be readily manually removed by being slid upwardly and out of the cavity along the inclined ramp surface of the cavity.

JP-A-1279222 is similar to US-A-4691820 but does not show a ramp. In this case the package includes a plastic base portion incorporating two molded cavities.

Although the foregoing blister package construction for the containment of contact lenses clearly provides a significant advance over prior structures requiring glass bottles and removable stopper arrangements for housing the contact lenses, there is significant scope for further improvements.

EP-A-0604177 which claims priority before, but which was published after the filing date of the present application. improves upon the uses and versatility of blister package construction in that the cavity is essentially of a semispherical configuration dimensioned so as to be adapted to closely support the contact lens therein immersed in an aqueous solution for ease of removal and also to facilitate an inspection process. Moreover, the foregoing construction primarily considers the utilization of such blister packages for the dispensing of individual contact lenses, with such blister packages being ordinarily separate or single packagings, which may then be housed in larger quantities in a further container, such as a rigid cardboard or paperboard carton of usual construction employed for the retail sales of the lenses.

Accordingly, it is an important aspect to be able to furnish a user of such disposable hydrophilic contact lenses with a specific supply of contact lenses, the latter of which are normally worn for only a single day; in essence, for ordinarily 8 to 18 hours within a 24-hour period and thereafter discarded. Hereby, the packaging of a supply of contact lenses should enable the user to store and provide indication for replenishing the supply of contact lenses at regular intervals; for example, at periods of 30 days. Consequently, the present invention contemplates the provision of packaging arrangements for specified quantities of such hydrophilic contact lenses, wherein these packaging arrangements are boxed in a carton enabling a rapid and precise determination as to the quantity of hydrophilic contact lenses contained therein, and with such packaging arrangements being of a compact nature which is completely protective of the hydrophilic lenses.

### SUMMARY OF THE INVENTION

In essence, the inventive concept pertains to packaging arrangements in which a plurality of blister packages each having a semi-circular cavity containing respectively one hydrophilic contact lens in a sterile aqueous solution. A specified quantity of such blister packages has molded plastic base members thereof each containing a contact lens positioned in a contiguous array, and is covered by a single flexible cover sheet constituted of a laminated foil or silicon oxide, or other suitable material structure to provide a sealed environment for each of the contact lenses contained in the cavity formed in each base member. Weakening lines are formed in the flexible cover sheet intermediate adjoining base members to enable detachment from the array of individual blister packages containing one of the hydrophilic contact lenses as may be required by a user. In particular, a plurality of such arrays of continuous packaging arrangements for contact lenses, which arrays are in an interconnected planar form, are adapted to be arranged superimposed in a generally rectangular carton. Each successively superimposed array is inverted and rotationally reversed relative to a preceding underlying array so as to enable the respective arrays to be interleaved and compactly support each other. The cavities containing the contact lenses of a superimposed array are arranged inverted relative to the cavities of an array of blister packages located therebeneath or thereabove, such that the mutually inverted cavities will be positioned adjacent to cavities of a superimposed array in an interleaved compact arrangement at minimum spacial requirements. Consequently, a plurality of planar arrays of blister packages which are each respectively interconnected by a single flexible cover sheet for each array are in a superimposed contacting relationship within a substantially rigid rectangular carton, with such arrays containing a specific quantity of disposable hydrophilic contact lenses to furnish a user with a desired supply; for instance, thirty (30) hydrophilic contact lenses in six superimposed arrays of five blister packages each; in essence, a thirty-day supply of contact lenses.

Each of the blister package base members which has a rectangular outstanding planar flange encompassing a respective cavity therein, the latter of which is offset towards one edge, includes a depending wall portion formed at the opposite edge of the flange so as to provide a support for a superimposed or therebeneath located array of blister packages, thereby formulating rigidly supported and compact packaging arrangements within the carton, in which the cavities containing the hydrophilic contact lenses of superimposed arrays are substantially protected against potentially damaging external influences, such as shocks or impacts which may be imparted to the filled carton during handling thereof.

Each molded plastic base member of a blister package may be constituted from a suitable injection molded or thermoformed thermoplastic sheet material, such as a polyolefin, for instance polypropylene; whereas the flexible cover sheet may be constituted of a laminate of a polypropylene film and aluminum foil or a layer of silicon oxide, suitably imprinted and which is adapted to be heat-sealed to the flange extending about the cavity of the package containing the hydrophilic contact lens. The flexible cover sheet may be of a construction and imprinted in a novel manner as disclosed in EP-A-0646471.

Accordingly, it is an object of the present invention to provide a packaging arrangement for hydrophilic contact lenses, wherein a plurality of lenses are located in base members of blister packages which are interconnected by a common flexible cover sheet to form an array of such packages.

A more specific object of the invention is to provide a packaging arrangement for a plurality of hydrophilic contact lenses in which a plurality of blister packages each having a cavity containing one of the contact lenses in a sterile aqueous solution are interconnected in an array by a single flexible cover sheet containing weakening lines intermediate the base members of the packages enabling separation of individual of the blister packages from the array for dispensing the contact lens from the separated package.

Another object of the present invention is to provide an arrangement for the storage of a plurality of superimposed arrays of blister packages within a substantially rigid carton structure.

Yet another object of the present invention is to provide structure for blister packages of the type described for the storage of hydrophilic contact lenses in a sterile aqueous environment, wherein pluralities of arrays of blister packages are arranged in specified mutually inverted superimposed relationship with a carton, and incorporate integrally formed supporting structure so as to be compactly and protectively supported within the carton.

### BRIEF DESCRIPTION OF THE DRAWINGS

Reference may now be had to the following detailed description of a preferred embodiment of a packaging arrangement for contact lenses which is constructed pursuant to the invention, taken in conjunction with the accompanying drawings; in which:
Figure 1 illustrates a perspective view of a rectangularly-shaped carton for the containment of a plurality of superimposed arrays of packaging arrangements for a specific quantity of contact lenses;
Figure 2 illustrates a sectional view taken along line 2 - 2 in Fig. 1;
Figure 3 illustrates a single array of a plurality of detachably interconnected blister packages each containing respectively one contact lens immersed in a sterile aqueous solution;
Figure 4 illustrates a perspective view of one of the blister packages shown as having been separated from the array of Fig. 3;
Figure 5 illustrates the base member of the blister package of Fig. 4 with the sealing cover sheet of the package having been stripped off so as to facilitate access to a contact lens contained in a cavity formed in the base member of the blister package;
Figure 6 illustrates a top plan view of the base member of the blister package shown in Fig. 5;
Figure 7 illustrates a bottom plan view of the base member of the blister package of Fig. 5;
Figure 8 illustrates a sectional view taken along line 8 - 8 in Fig. 6; and
Figure 9 illustrates a sectional view taken along line 9 - 9 in Fig. 6.

### DETAILED DESCRIPTION OF A PREFERRED EMBODIMENT

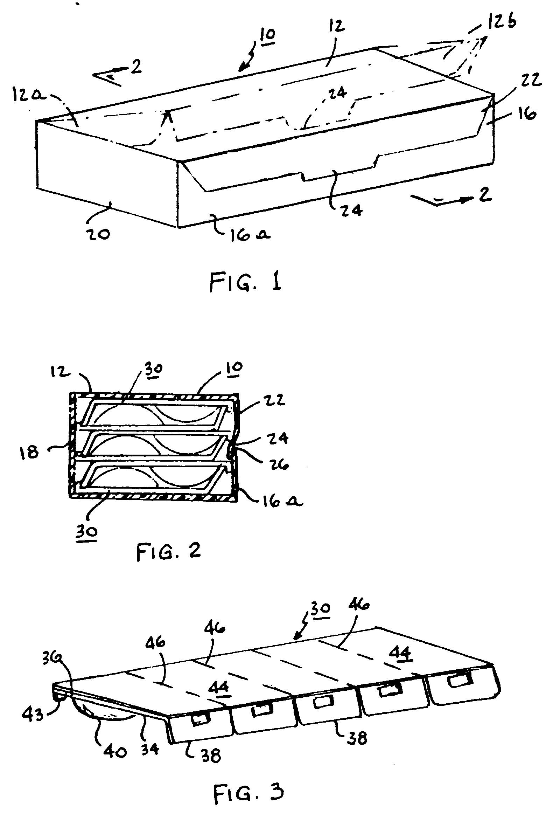

Referring now in more specific detail to the drawings, and in particular to Figs. 1 and 2, there is illustrated a generally rectangular carton 10 which is adapted to receive a plurality of planar arrays of packaging arrangements for the sealed containment of contact lenses, especially disposable hydrophilic contact lenses, as described in more specific detail hereinbelow.

The rectangular carton 10, which is preferably constituted of paperboard, includes flat top and bottom wall panels 12 and 14, and front and rear walls or panels 16 and, respectively, 18 which are adapted to be closed by being folded from a carton blank along suitable fold lines, and opposite ends 20; (only one shown) through an adhesive or glued construction as is known in the carton forming technology. The top wall panel 12 is adapted to be swung upwardly about a rear hinge line, as shown by the phantom illustration, to open the carton 10, and includes side flaps 12a, 12b which may be tucked within the confines of the carton beneath the end walls 20. The front wall panel 16 includes a lower portion 16a which is adapted to be glued to the end walls 20 by means of end flaps (not shown). The upper wall panel 12 includes a downwardly depending front flap 22 having a centrally located latching tab 24 at a lower edge thereof which is adapted to be tucked into a cooperating latching slit 26 centrally formed in the lower front wall panel 16 to facilitate reclosing of the carton, as shown in Fig. 2.

The carton 10, as is known in the art, may be equipped with a decorative glossy or semi-glossy exterior surface, which may be imparted with suitable single or multi-colored imprinting and/or embossing representative of the product contained therein, identifying legends and logos pertaining to the company manufacturing and/or marketing the product, instructions pertaining to the use of the product packaged in the carton, and other suitable legends, decorative indicia and the like.

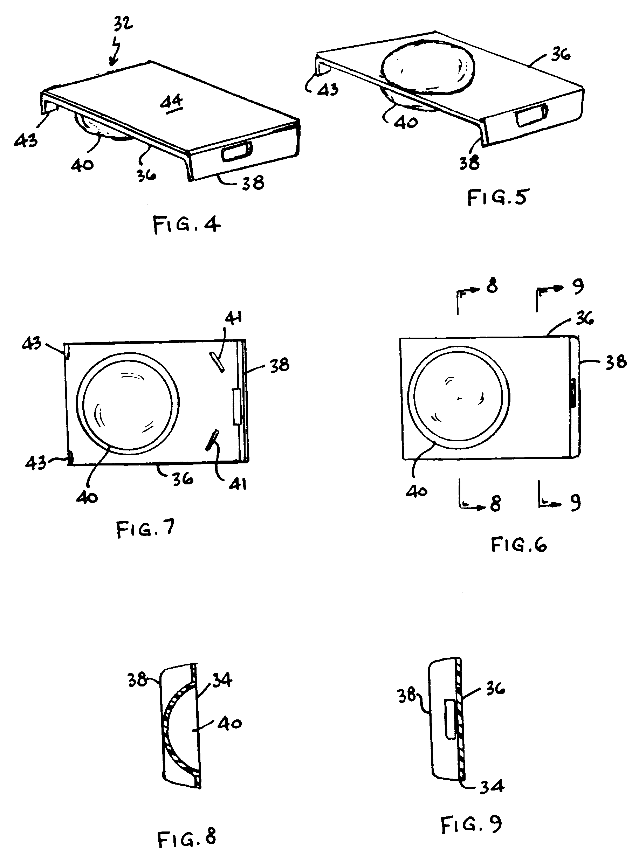

Within the carton 10 there is adapted to be housed a plurality of arrays of packaging arrangements 30, as is more specifically illustrated in Fig. 3 of the drawings, and which essentially represent the inventive concept for the packaging of desired quantities of individually dispensable contact lenses.

In essence, Fig. 3 illustrates the array of packaging arrangements 30 as consisting of a plurality of interconnected blister packages 32, wherein each blister package 32, as represented in further detail in Figs. 4 through 9 of the drawings, includes a base member 34 consisting of a planar essentially rectangularly-shaped flange 36 having an integral depending wall portion 38 at one edge thereof. Offset towards an opposite edge 39 of the flange 36 a cavity 40 is formed therein which is of an essentially semispherical configuration, generally in conformance with the curvilinear shape of a contact lens (not shown), adapted to be stored therein in a sealed condition while immersed in a suitable sterile aqueous solution. However, other cavity configurations also readily lend themselves to the invention, such as semi-spherical, oval, or the like. The height of the wall portion 38 depending from the planar flange 36 is somewhat analogous to the height or depth of the cavity 40 containing the contact lens, as can be ascertained more clearly from Figs. 2 and 8 of the drawings. Formed in the surface of the flange 36 proximate the depending wall portion 38 are a plurality of generally "chevron-shaped" raised ridges 41 which will assist in supporting the cavity structure of a superimposed inverted blister package 32 when positioned in the carton 10. Similarly the edge of the flange 36 opposite that possessing the depending wall portion is also provided with depending protuberances 43 which will also aid in positioning the blister packages in the carton, as described in detail hereinbelow.

The base member 34 of each so-called blister package is constituted from an injection-molded or thermoformed plastic sheet material; for instance, such as polypropylene, in a manner similar to that described in U.S. Patent No. 4,691,820 to Martinez; which is assigned to the common assignee of the present application, and the disclosure of which is incorporated herein by reference.

Positioned to extend over the base members 34 of a plurality of blister packages 32, in this case forming an array of five (5), is a continuous flexible cover sheet 44, as shown in Fig. 3, having a series of parallel spaced weakening lines 46, such as perforations, discontinuous slits or the like, provided between each of the adjacently located base members 34, to enable suitable detachment from the array of individual or single blister packages 32 each containing a single contact lens, as shown in Fig. 4, in accordance with the need of a user. The flexible cover sheet 44 is adhesively fastened to suitable regions of the surface of the flanges 36 facing the cover sheet, such as by heat sealing, ultrasonic sealing, adhesives or other acceptable methods, so as to at least encompass each cavity 40 containing a contact lens immersed in the sterile aqueous solution, and to provide a sealing containment for each contact lens in its respective cavity. Other sealing locations may also be provided at locations as desired between the surface of the flexible cover sheet 44 facing the surface of the flange 36 so as to provide adequate regions of adherence therewith, while permitting various edge portions between these components 36, 44 to remain unattached to facilitate a finger-gripping engagement for separating the severed cover sheet portion from the detached base member 34 in order to gain access to the contact lens which is contained in the applicable cavity 40 thereof.

The flexible cover sheet 44 is preferably constituted of a laminated foil, silicon oxide or other suitable material structure possessing a polypropylene film, or possibly a PET film, on at least one external surface thereof adapted to contact the facing surface of the flange 36 so as to enable adhesive or heat-sealing therewith, as mentioned hereinabove. The laminated foil constituting the flexible cover sheet 32 may be of a multi-layered construction having suitable double-sided imprinting provided thereon, for example, as disclosed in European Patent Application No. 94305972.5.

As disclosed herein, the inventive packaging arrangement is adapted to provide for a packaged supply of disposable hydrophilic contact lenses, each of which is intended to be used for only one day and then discarded, for a period of 30 days; in effect, requiring the carton 10 to be able to store 30 contact lenses, each in a sterile sealed condition, in each one of the cavities of the blister packages 32 so as to be individually dispensable.

Hereby, as shown in Fig. 3, an array of blister packages 30 includes five base members 34, each having respectively one cavity 40 containing a single contact lens, the base members 34 being collectively covered and sealingly contacted by the single continuous flexible cover sheet 44, with the latter scored along the spaced weakening lines 46 to enable sequential separation of the array into five individual blister packages 32, as required by the needs of a user of the contact lenses.

In order to provide for the compact and protective housing within the carton 10 of the thirty (30) blister packages 32 each containing a single contact lens within a respective sealed cavity 40, as can be ascertained from Fig. 2 of the drawings, six arrays 30 each respectively consisting five blister packages 32 joined by a common cover sheet 44, are superimposed in mutually inverted and reversed nesting positions such that the cavities 40 of an array 30 are adjacent to the cavities 40 of an inverted array 30 located therebeneath or thereabove, as may be the case. The upstanding end wall 38 of the flange 36 of each base member 34 of the blister packages 32, in cooperation with the depending end wall 38 of a therewith nested inverted array 30, as shown in Fig. 2, will form a support structure which is protective of the cavities 40 containing the contact lenses. Assisting this supportive and protective effect are the raised "chevron-shaped" ridges 41 which contact and contribute to the positioning in place of the cavities 40 of superimposed blister packages 32 adjacent the cavities of the arrays 30 beneath or above they are located. Similarly, the locating of the protuberances 43 of the flanges 36 adjacent to the depending wall portions 38 of superimposed or therebeneath located arrays of blister packages will also inhibit lateral displacements between the arrays. This arrangement of the arrays 30, in conjunction with the enclosing wall structure of the carton 10, provides for a compact and highly protective positioning of the various arrays 30 of contact lenses in their packaging arrangements within the carton 10.

In order to ensure that there is encountered only a minimal limited displacement and internal shifting of the contents of the carton, the preferable external peripheral rectangular dimensions of each array 30 of five blister packages are configure to be in close conformance with the internal rectangular dimensions or space within the carton 10 such that the front and end wall panels 16, 18, the end wall panels 20 and the top and bottom walls 12 and 14 of the carton 10 provide additional supporting structure maintaining the arrays 30 of blister packages in generally fixed and stationary positions within the carton, even upon successive withdrawals of individual of the blister packages 32 over a period of time by a practitioner or user of the contact lenses.

From the foregoing, it becomes readily apparent to one of skill in the art that the present invention provides for a simple packaging of predetermined quantities of blister packages provided in packaging arrangements and units adapted to be compactly stored within a carton in superimposed relationship which not only provides for a novel packaging but for added support and protection for each of the blister packages and the contact lenses contained in the cavities thereof.

Although the foregoing has been described with arrays 30 providing for collectively thirty contact lenses, other quantities may be considered to fall within the purview of the invention; i.e. such as 5, 10, 15, 20, or other numbers of packaging arrangements.

While there has been shown and described what is considered to be a preferred embodiment of the invention, it will, of course, be understood that various modifications and changes in form or detail could readily be made without departing from the spirit of the invention. It is, therefore, intended that the invention be not limited to the exact form and detail herein shown and described, nor to anything less than the whole of the invention herein disclosed as hereinafter claimed.

## Claims

1. A packaging arrangement (30) for the sealed containment of at least two hydrophilic contact lens in a sterile aqueous solution, comprising:
a) a plurality of molded plastic base members (34), each having a cavity (40) for containing a contact lens immersed in said solution, said base members (34) each including a flange (36) extending outwardly about the periphery of said cavity (40), said cavity (40) consisting of a generally curvilinear concave indentation in said base member (34) and having a shape in substantial conformance with the shape of the contact lens adapted to be contained therein; and
b) a flexible cover sheet (44) superimposed over said contiguously arranged base members (34) in order to interconnect them in an array, and dimensioned to be detachably sealed to said flanges (36), said cover sheet (44) sealingly extending about said cavities (40) and having unsealed edge portions providing gripping means for enabling separating said cover sheet (44) from said flange (36) so as to expose a cavity (40) and facilitating external access to the contact lens, and weakening lines (46) being formed in said flexible cover sheet (44) intermediate each of said base members (34) to enable separating said plurality of base members (34) into individual packaging each having a single base member.

2. The packaging arrangement (30) of claim 1, wherein said base members (34) include flaps, extending from said flanges (36), engageable in cooperation with gripping of said gripping means for facilitating external access to a contact lens.

3. The packaging arrangement (30) of claim 1 or claim 2, wherein said flanges (36) include a raised seal area on their surfaces.

4. The packaging arrangement (30) of any one of claims 1 to 3, wherein said flanges (36) have a generally planar surface configuration facing said superimposed cover sheet in (44) in contacting relationship.

5. The packaging arrangement (30) of claim 4, wherein a seal is formed between said cover sheet (44) and the planar surface of said flanges (36) sealingly encompassing the peripheral edge of said cavities (40).

6. The packaging arrangement (30) of claim 5, wherein said seal comprises a heat seal.

7. The packaging arrangement (30) of any one of claims 1 to 6, wherein said flanges (36) have depending wall portions (38) at one end extending in a direction away from said superimposed cover sheet (44).

8. The packaging arrangement of claim 7, wherein protuberances (43) are formed on an end of said flanges (36) opposite the end having said depending wall portions (38), said protuberances (43) extending in generally the direction away from said superimposed cover sheet (44).

9. The packaging arrangement (30) of claim 7, wherein raised ridges are formed in the surface of said flanges (36) opposite said planar surfaces proximate said depending wall portions (38).

10. The packaging arrangement (30) of claim 9, wherein said ridges comprise a plurality of spaced chevron-shaped ridges (41).

11. The packaging arrangement (30) of any one of claims 7 to 10, wherein said flanges (36) are rectangular and said depending wall portions (38) are integrally formed with said flanges (36) at one end edge of said flanges (36).

12. The packaging arrangement (30) of any one of claims 1 to 11, wherein said molded plastic base members (34) are constituted of a thermoformable polymer material.

13. The packaging arrangement (30) of any one of claims 1 to 12, wherein said flexible cover sheet is a multilayered laminate having an outer layer of said laminate consisting of a polypropylene film contacting the surface of the flanges (36) of said molded plastic base members (34).

14. The packaging arrangement (30) of claim 13, wherein said polypropylene film is heat sealed to said flange surfaces (36) of the molded plastic base members (34) for sealing the cavities (40) containing the contact lenses.

15. The packaging arrangement (30) of claim 13 or claim 14, wherein said flexible cover sheet (44) comprises a foil laminate.

16. The packaging arrangement (30) of any one of claims 13 to 15, wherein said flexible cover sheet (44) comprises a silicon oxide barrier layer intermediate said outer plastic film layer.

17. The packaging arrangement (30) of any one of claims 1 to 16, wherein said molded plastic base members (34) and said flexible cover (44) conjointly form a moisture and vapor-imperviously sealed containment for said contact lenses in said cavities (40).

18. The packaging arrangement (30) of any one of claims 1 to 17, wherein said base members (34) are interconnected in the array (30) by said flexible cover sheet (44) for the containment of a specified number of said contact lenses arranged one each in each cavity (40) of each of said base members (34).

19. The packaging arrangement (30) of claim 18, wherein said flexible cover sheet (44) extends over said plurality of base members (34) and is sealingly connected to each said flange (36) of respectively each said base member (34).

20. The packaging arrangement (30) of any preceding claim, wherein said weakening lines (46) in said flexible cover sheet (44) comprise perforations extending at least partially through said cover sheet (44).

21. The packaging arrangement (30) of any preceding claim, wherein said flexible cover sheet (44) connects said plurality of base members (34) in a coplanar array.

22. The packaging arrangement (30) of claim 21, wherein said array of base members (34) comprises linearly arranged base members (34), said flexible cover sheet (44) having a substantially rectangular configuration.

23. The packaging arrangement (30) of any preceding claim, wherein said plurality of base members (34) are separable into individual blister packages (32) each containing a single contact lens.

24. The packaging arrangement (30) of any preceding claim further comprising a generally rigid self-supporting carton (10) for containment of a plurality of said arrays.

25. The packaging arrangement (30) of claim 24, wherein said carton (10) has a generally rectangular configuration.

26. The packaging arrangement (30) of claim 24 or claim 25, wherein said carton (10) comprises front and back wall panels (16,18), top and bottom wall panels (12,14) connected to said front and back wall panels (16,18), and end wall panels and flap structure being fastened to respectively each other and to said front, back, top and bottom wall panels (12,14,16,18) to form a closed carton structure, said front wall panel (16) and top wall panel (12) including openable upper and lower flap portions to enable opening said carton (10) and mutually cooperable latching structure for relatching said carton (10) in a closed position.

27. The packaging arrangement (30) of any one of claims 24 to 26, wherein said carton (10) is constituted of paperboard.

28. The packaging arrangement (30) of any one of claims 24 to 27, wherein indicia and content-identifying legends are imprinted on at least one of the exterior surfaces of said carton (10).

29. The packaging arrangement (30) of any one of claims 24 to 28, wherein said plurality of arrays are arranged in superimposed mutually inverted nested relationship.

30. The packaging arrangement (30) of claim 29, wherein each cavity (40) of each said molded plastic base member (34) is offset towards one edge of said flange (36), said superimposed arrays being inverted with respect of the orientation of each other said array to enable the compact nested positioning thereof within said carton (10).

31. The packaging arrangement (30) of claim 30, wherein each said flange (36) has a rectangular configuration wherein the depending wall portion (38) is located along an edge distant from the edge towards which each said cavity (40) is offset, said depending wall portion (38), being of a height substantially commensurate with the height of an indentation in each base member (34) forming each cavity (40).

## Patentansprüche

1. Verpackungsanordnung (30) zur dichten Aufbewahrung von mindestens zwei hydrophilen Kontaktlinsen in einer sterilen wäßrigen Lösung umfassend:
a) eine Vielzahl geformter Kunststoff-Grundkörper (34), von denen jeder eine Vertiefung (40) zur Aufnahme einer in die genannte Lösung eingetauchten Kontaktlinse aufweist, wobei die Grundkörper (34) jeweils einen Flansch (36) aufweisen, der sich rund um den Außenumfang der Vertiefung (40) nach außen erstreckt und die Vertiefung (40) aus einer allgemein konkav gekrümmten Einprägung im Grundkörper (34) besteht und eine Form hat, die im wesentlichen der Form der darin aufzubewahrenden Kontaktlinse entspricht und
b) eine flexible Abdeckfolie (44), welche durchgehend auf die Grundkörper (34) aufgelegt ist, um diese in einer Anordnung untereinander zu verbinden, und derart bemessen ist, daß sie dicht aber entfernbar mit den Flanschen (36) verbunden ist, wobei die Abdeckfolie (44) die Vertiefungen (40) dicht verschließt und Kantenbereiche hat, welche nicht dicht verschlossen sind, um das Abtrennen der Abdeckfolie (44) vom Flansch (36) zu ermöglichen und dadurch eine Vertiefung (40) freizugeben und den Zugang zur Kontaktlinse von außen zu ermöglichen und weiterhin in der flexiblen Abdeckfolie (44) zwischen den Grundkörpern (34) Bruchlinien (46) angebracht sind, um die Vielzahl der Grundkörper (34) in Einzelverpakkungen trennen zu können, deren jede einen einzelnen Grundkörper (34) umfaßt.

2. Verpackungsanordnung (30) nach Anspruch 1, bei welcher die Grundkörper (34) Zungen aufweisen, die sich jeweils von den Flanschen (36) aus erstrecken und im Zusammenwirken mit der Greifeinrichtung den Zugang zu einer Kontaktlinse von außen erleichtern.

3. Verpackungsanordnung (30) nach Anspruch 1 oder 2, bei welcher die Flansche (36) einen erhöhten Abdichtbereich auf iher Fläche aufweisen.

4. Verpackungsanordnung (30) nach einem der Ansprüche 1 bis 3, bei welcher die Flansche (36) eine allgemein ebene Oberfläche haben, welche der aufgelegten Abdeckfolie (44) zugewandt ist und mit dieser Kontakt hat.

5. Verpackungsanordnung (30) nach Anspruch 4, bei welcher zwischen der Abdeckfolie (44) und der ebenen Oberfläche derFlansche (36) eine Abdichtung ausgebildet ist, welche die Außenumfangskante der Vertiefungen (40) abdichtet.

6. Verpackungsanordnung (30) nach Anspruch 5, bei welcher die Abdichtung mittels Wärme erfolgt.

7. Verpackungsanordnung (30) nach einem der Ansprüche 1 bis 6, bei welcher die Flansche (36) an einem Ende herabreichende Wandabschnitte (38) aufweisen, welche sich in einer Richtung von der aufgelegten Abdeckfolie (44) weg erstrecken.

8. Verpackungsanordnung (30) nach Anspruch 7, bei welcher an einem Ende der Flansche (36), das dem Ende mit dem herabreichenden Wandabschnitt gegenüberliegt, Vorsprünge (43) ausgebildet sind, welche sich allgemein in einer Richtung von der aufgelegten Abdeckfolie (44) weg erstrecken.

9. Verpackungsanordnung (30) nach Anspruch 7, bei welcher auf der Oberfläche der Flansche (36), welche den ebenen Oberflächen gegenüber liegt, in der Nähe der herabreichenden Wandabschnitte (38) erhabene Leisten ausgebildet sind.

10. Verpackungsanordnung (30) nach Anspruch 9, bei welcher die Leisten eine Vielzahl im Abstand voneinander angeordnete, V-förmige Leisten (41) sind.

11. Verpackungsanordnung (30) nach einem der Ansprüche 7 bis 10, bei welcher die Flansche (36) rechteckig und die herabreichenden Wandbereiche (38) an einer Kante der Flansche (36) integriert angeformt sind.

12. Verpackungsanordnung (30) nach einem der Ansprüche 1 bis 11, bei welcher die geformten Kunststoff-Grundkörper (34) aus einem thermoplastischen Polymermaterial bestehen.

13. Verpackungsanordnung (30) nach einem der Ansprüche 1 bis 12, bei welcher die flexible Abdeckfolie (44) ein Mehrlagen-Laminat ist, wobei eine Außenlage des Laminates eine Polypropylenfolie ist, welche mit der Oberfläche der Flansche (36) der geformten Kunststoff-Grundkörper (34) Kontakt hat.

14. Verpackungsanordnung (30) nach Anspruch 13, bei welcher die Polypropylenfolie mittels Wärme dicht mit den Flanschflächen der geformten Kunststoff-Grundkörper (34) verbunden ist, um die Vertiefungen (40), welche die Kontaktlinsen enthalten, abzudichten.

15. Verpackungsanordnung (30) nach einem der Ansprüche 13 oder 14, bei welcher die flexible Abdeckfolie (44) ein Folienlaminat ist.

16. Verpackungsanordnung (30) nach einem der Ansprüche 13 bis 15, bei welcher die flexible Abdeckfolie (44) eine Siliziumoxid-Barriereschicht zwischen äußeren Kunststoffolien umfaßt.

17. Verpackungsanordnung (30) nach einem der Ansprüche 1 bis 16, bei welcher die geformten Kunststoff -Grundkörper (34) und die fexible Abdeckfolie (44) zusammen einen feuchtigkeits- und dampfundurchlässigen Behälter für die Kontaktlinsen in den Vertiefungen (40) bilden.

18. Verpackungsanordnung (30) nach einem der Ansprüche 1 bis 17, bei welcher die Grundkörper (34) in der Anordnung (30) durch die flexible Abdeckfolie (44) verbunden sind, um einen Behälter für eine bestimmte Anzahl von Kontaktlinsen zu bilden, von denen jeweils eine in jeder Vertiefung (40) jedes Grundkörpers (34) enthalten ist.

19. Verpackungsanordnung (30) nach Anspruch 18, bei welcher sich die flexible Abdeckfolie (44) über eine Vielzahl von Grundkörpern (34) erstreckt und mit jedem Flansch (36) eines jeden Grundkörpers (34) dicht verbunden ist.

20. Verpackungsanordnung (30) nach einem der bisherigen Ansprüche, bei welcher die Bruchlinien (46) der flexiblen Abdeckfolie (44) Perforationen sind, die sich zumindest teilweise über die Abdeckfolie (44) erstrecken.

21. Verpackungsanordnung (30) nach einem der bisherigen Ansprüche, bei welcher die flexible Abdeckfolie (44) eine Vielzahl von Grundkörpern (34) zu einer koplanaren Anordnung verbindet.

22. Verpackungsanordnung (30) nach Anspruch 21, bei welcher die Anordnung von Grundkörpern (34) linear angeordnete Grundkörper (34) umfaßt und die flexible Abdeckfolie (44) im wesentlichen Rechteckform hat.

23. Verpackungsanordnung (30) nach einem der bisherigen Ansprüche, bei welcher die Vielzahl der Grundkörper (34) in einzelne Blisterverpackungen (23) teilbar ist, von denen jede eine einzelne Kontaktlinse enthält.

24. Verpackungsanordnung (30) nach einem der bisherigen Ansprüche, welche weiterhin einen im allgemeinen steifen, selbsttragenden Karton (10) zur Aufnahme einer Vielzahl der Anordnungen umfaßt.

25. Verpackungsanordnung (30) nach Anspruch 24, bei welcher der Karton (10) eine allgemein rechteckige Form hat.

26. Verpackungsanordnung (30) nach Anspruch 24 oder 25, bei welcher der Karton (10) eine Vorder- und eine Rückwand (16, 18), die durch eine Deckel- und Bodenwand (12, 14) verbunden sind sowie Stirnwände mit einer Zungenanordnung umfaßt, die jeweils an Vorder- oder Rückwand, Deckel- oder Bodenwand (12, 14, 16, 18) befestigt sind, um dadurch eine geschlossene Kartonanordnung zu bilden, wobei die Vorderwand (16) und die Deckelwand (12) zu öffnende obere und untere Zungenabschnitte aufweisen, um den Karton (10) durch gegenseitiges Zusammenwirken einer Rastanordnung öffnen und auch wieder verschließen zu können.

27. Verpackungsanordnung (30) nach einem der Ansprüche 24 bis 26, bei welcher der Karton (10) aus Pappe besteht.

28. Verpackungsanordnung (30) nach einem der Ansprüche 24 bis 27, bei welcher Kennzahlen und den Inhalt identifizierende Angaben auf mindestens eine der Außenflächen des Kartons (10) aufgedruckt sind.

29. Verpackungsanordnung (30) nach einem der Ansprüche 24 bis 28, bei welcher eine Vielzahl von Anordnungen alternierend übereinander gestapelt angeordnet ist.

30. Verpackungsanordnung (30) nach Anspruch 29, bei welcher jede Vertiefung (40) eines jeden geformten Kunststoff-Grundkörpers (34) zu einer Kante des Flansches (36) hin versetzt ist, und die übereinander angeordneten Anordnungen in bezug auf ihre Ausrichtung einander abwechseln, so daß eine kompakte Positionierung im Karton (10) möglich ist.

31. Verpackungsanordnung (30) nach Anspruch 30, bei welcher jeder Flansch (36) eine Rechteckform hat, wobei sich der herabreichende Wandbereich (38) an derjenigen Kante befindet, die von der Kante, zu der hin die Vertiefung (40) versetzt ist, abgewandt ist, wobei die Höhe des Wandbereiches (38) im wesentlichen der Höhe der Einprägung entspricht, welche in jedem Grundkörper (34) die Vertiefung (40) bildet.

## Revendications

1. Dispositif d'emballage (30) pour le logement scellé d'au moins deux lentilles de contact hydrophiles dans une solution aqueuse stérile, comprenant :
a) une pluralité d'éléments de base (34) en matière plastique moulée, chaque élément de base ayant une cavité (40) pour contenir une lentille de contact immergée dans ladite solution, lesdits éléments de base (34) comprenant chacun une partie centrale (36) s'étendant vers l'extérieur autour de la périphérie de ladite cavité (40), ladite cavité (40) se composant d'une indentation concave généralement curviligne dans ledit élément de base (34) et ayant une forme pratiquement en conformité avec la forme de la lentille de contact adaptée pour y être logée à l'intérieur; et
b) une feuille de protection flexible (44) superposée par-dessus lesdits éléments de base (34) disposés de façon contiguë de manière à les relier l'un à l'autre en un ensemble et dimensionnée pour être scellée de façon détachable sur lesdites parties centrales (36), ladite feuille de protection (44) s'étendant en étant scellée autour desdites cavités (40) et en ayant des parties du bord non scellées fournissant un moyen de préhension pour permettre de séparer ladite feuille de protection (44) de ladite partie centrale (36) de façon à exposer une cavité (40), et facilitant l'accès extérieur à la lentille de contact, et comprenart des lignes de faiblesse (46) formées dans ladite feuille de protection flexible (44) entre chacun desdits éléments de base (34) pour permettre de séparer les éléments de base (34) formant ladite pluralité d'éléments, en un emballage individuel ayant chacun un seul élément de base.

2. Dispositif d'emballage (30) selon la revendication 1 dans lequel lesdits éléments de base (34) comprennent des rabats s'étendant à partir desdites parties centrales (36) et pouvant être engagés en coopération de prise dudit moyen de préhension pour faciliter l'accès extéreur à une lentille de contact

3. Dispositif d'emballage (30) selon la revendication 1 ou 2, dans lequel lesdites parties centrales (36) comprennent une zone en relief formant joint sur leurs surfaces.

4. Dispositif d'emballage (30) selon l'une quelconque des revendications 1 à 3, dans lequel lesdites parties centrales (36) ont une configuration de surface généralement plane faisant face à ladite feuille de protection superposée (44) avec laquelle elle est en contact.

5. Dispositif d'emballage (30) selon la revendication 4, dans lequel un joint est formé entre ladite feuille de protection (44) et la surface plane desdites parties centrales (36) englobant de façon scellée le bord périphérique desdites cavités (40)

6. Dispositif d'emballage (30) selon la revendication 5, dans lequel ledit joint se compose d'un joint thermique.

7. Dispositif d'emballage (30) selon l'une quelconque des revendications 1 à 6, dans lequel lesdites parties centrales (36) ont des parties tombantes (38) formant paroi à une extrémité s'étendant dans une direction éloignée de ladite feuille de protection superposée, (44).

8. Dispositif d'emballage (30) selon la revendication 7, dans lequel des protubérances (43) sont formées sur une extrémité desdites parties centrales (36) à l'oppose' de l'extrémité ayant lesdites parties tombantes (38) formant paroi, lesdites protubérances (43) s'étendant dans la direction généralement éloignée de ladite feuille de protection superposée (44).

9. Dispositif d'emballage (30) selon la revendication 7, dans lequel des stries en relief sont formées à la surface desdites parties centrales (36) à l'opposé desdites surfaces planes, à proximité desdites parties tombantes (38) formant paroi.

10. Dispositif d'emballage (30) selon la revendication 9, dans lequel lesdites stries se composent d'une pluralité de stries (41) espacées et en forme de chevrons.

11. Dispositif d'emballage (30) selon l'une quelconque des revendications 7 à 10, dans lequel lesdites parties centrales (36) sont rectangulaires, lesdites parties tombantes (38) formant paroi étant formées solidairement avec lesdites parties centrales (36) au niveau d'un bord d'extrémité desdites parties centrales (36).

12. Dispositif d'emballage (30) selon l'une quelconque des revendications 1 à 11, dans lequel lesdits éléments de base (34) en matière plastique moulée sont constitués d'un matériau polymère thermoformable.

13. Dispositif d'emballage (30) selon l'une quelconque des revendications 1 à 12, dans lequel ladite feuille de protection flexible est un laminé multicouches ayant une couche extérieure dudit laminé se composant d'un film en polypropylène en contact avec la surface des parties centrales (36) desdits éléments de base (34) en matière plastique moulée.

14. Dispositif d'emballage (30) selon la revendication 13, dans lequel ledit film en polypropylène est thermocollé sur lesdites surfaces (36) des parties centrales des éléments de base (34) en matière plastique moulée pour sceller les cavités (40) contenant les lentilles de contact.

15. Dispositif d'emballage (30) selon la revendication 13 ou 14, dans lequel ladite feuille de protection flexible (44) comprend un laminé en feuilles.

16. Dispositif d'emballage (30) selon l'une quelconque des revendications 13 à 15, dans lequel ladite feuille de protection flexible (44) comprend une couche formant barrière en oxyde de silicium entre ladite couche extérieure du film en matière plastique.

17. Dispositif d'emballage (30) selon l'une quelconque des revendications 1 à 16, dans lequel lesdits éléments de base (34) en matière plastique moulée et ladite feuille de protection flexible (44) forment conjointement un logement scellé imperméable à l'humidité et à la vapeur pour lesdites lentilles de contact dans lesdites cavités (40).

18. Dispositif d'emballage (30) selon l'une quelconque des revendications 1 à 17, dans lequel lesdits éléments de base (34) sont reliés l'un à l'autre dans l'ensemble (30) par ladite feuille de protection flexible (44) pour le logement d'un nombre spécifié desdites lentilles de contact disposées chacune dans chacune des cavités (40) de chacun desdits éléments de base (34).

19. Dispositif d'emballage (30) selon la revendication 18, dans lequel ladite feuille de protection flexible (44) s'étend par-dessus ladite pluralité des éléments de base (34) et qui est reliée en étant scellée à chaque partie centrale (36) respectivement de chacun desdits éléments de base (34).

20. Dispositif d'emballage (30) selon l'une quelconque des revendications précédentes, dans lequel lesdites lignes de faiblesse (46) dans ladite feuille de protection flexible (44) comprennent des perforations s'étendant au moins partiellement à travers ladite feuille de protection (44).

21. Dispositif d'emballage (30) selon l'une quelconque des revendications précédentes, dans lequel ladite feuille de protection flexible (44) relie ladite pluralité d'élements de base (34) dans un ensemble coplanaire.

22. Dispositif d'emballage (30) selon la revendication 21, dans laquelle ledit ensemble d'éléments de base (34) comprend des éléments de base (34) agencés linéairement, ladite feuille de protection flexible (44) ayant une configuration pratiquement rectangulaire.

23. Dispositif d'emballage (30) selon l'une quelconque des revendications précédentes, dans lequel les éléments de base (34) formant ladite pluralité d'éléments sont séparables en blisters individuels (32) contenant chacun une seule lentille de contact.

24. Dispositif d'emballage (30) selon l'une quelconque des revendications précédentes comprenant en outre un carton (10) autonome généralement rigide pour contenir une pluralité desdits ensembles.

25. Dispositif d'emballage (30) selon la revendication 24, dans lequel ledit carton (10) a une configuration généralement rectangulaire.

26. Dispositif d'emballage (30) selon la revendication 24 ou 25, dans lequel ledit carton (10) comprend des panneaux avant et arrière (16, 18) formant paroi, des panneaux du dessus et du dessous (12, 14) formant paroi reliés auxdits panneaux avant et arrière (16, 18) formant paroi, et des panneaux d'extrémité formant paroi et une structure à rabats fixés respectivement les uns aux autres et auxdits panneaux avant, arrière, du dessus et du dessous (12, 14, 16, 18) formant Paroi pour constituer une structure fermée en carton, ledit panneau avant (16) formant paroi et ledit panneau du dessus (17) formant paroi comprenant des parties supérieures et inférieures à rabats pouvant être ouvertes pour permettre l'ouverture dudit carton (10) et une structure à panneaux se verrouillant mutuellement pour reverrouiller ledit carton (10) en position fermée.

27. Dispositif d'emballage (30) selon l'une quelconque des revendications 24 à 26, dans lequel ledit emballage (10) est en carton.

28. Dispositif d'emballage (30) selon l'une quelconque des revendications 24 à 27, dans lequel des marques et des indications identifiant le contenu sont imprimées sur au moins une des surfaces extérieures dudit carton (10).

29. Dispositif d'emballage (30) selon l'une quelconque des revendications 24 à 28, dans lequel ladite pluralité d'ensemble est disposée suivant un agencement d'ensemble superposés et emboîtés tête-bêche l'un par rapport à l'autre.

30. Dispositif d'emballage (30) selon la revendication 29, dans lequel chaque cavité (40) de chacun desdits éléments de base (34) en matière plastique moulée est décalée vers un bord de ladite partie centrale (36), lesdits ensembles superposés étant inversés dans leur orientation l'un par rapport à l'autre pour pouvoir les emboîter de façon compacte à l'intérieur dudit carton (10).

31. Dispositif d'emballage (30) selon la revendication 30, dans lequel chacune desdites parties centrales (36) a une configuration rectangulaire où la partie tombante (38) formant paroi est placée le long d'un bord distant du bord vers lequel chacune desdites cavités (40) est décalée, ladite partie tombante (38) formant paroi étant d'une hauteur pratiquement proportionnelle à la hauteur d'une indentation dans chacun des éléments de base (34) formant chaque cavité (40).
